# EUROPEAN PATENT APPLICATION

(11) **EP 4 099 014 A1**
(43) Date of publication of application: **07.12.2022**
(21) Application number: 21177047.4
(22) Date of filing: 01.06.2021
(51) Int. Cl.: G01N 33/50, B01L 3/00, G01N 33/543, G01N 33/574

(54) **RAPID CAPTURE OF TUMOR-ASSOCIATED EXTRACELLULAR VESICLES BY LIPID PATCH MICROARRAYS**

(71) Applicant: Westfälische Wilhelms-Universität Münster, 48149 Münster (DE)
(72) Inventor: Liu, Hui-Yu, 76135 Karlsruhe (DE); Sekula-Neuner, Sylwia, 76137 Karlsruhe (DE); Nazarenko, Irina, 79286 Glottertal (DE); Fuchs, Harald, 48145 Münster (DE); Hirtz, Michael, 48653 Coesfeld (DE)
(74) Representative: Lahrtz, Fritz

(57) **Abstract**

The present invention relates to a method for isolating extracellular vesicles (EVs) from a sample, comprising the step of contacting the sample with a supported lipid membrane (SLM) capable of binding the EVs.

## Description

The present invention relates to a method for isolating extracellular vesicles (EVs) from a sample as well as to microfluidic chips, which can be used in that method. The invention further relates to a method for diagnosing tumors.

### BRIEF DESCRIPTION OF RELATED ART

Extracellular vesicles (EVs) are lipid-bilayer enclosed structures shed by nearly all cells of a human body, containing abundant information about the cell of origin in form of proteins, RNA and DNA. They play an important role for cell-cell communication and present in all body fluids, like blood, urine, milk, tears, and others (H. Kang, 2017, Micro Nano Syst. Lett.; C. Théry, 2018, J. Extracell). EVs are highly heterogeneous, and can be divided based on their origin into exosomes, microvesicles, and apoptotic bodies (C. Théry, 2018, J. Extracell; M. Yáñez-Mó, 2015, J. Extracell. Vesicles). Exosomes are small EVs of 40-100 nm diameter released by the back-fusion of multivesicular bodies (MVB) with the plasma membrane (C. V. Harding, 2013, J. Cell Biol; B. T. Pan, 1985, J. Cell Biol). Microvesicles are a heterogeneous population of EVs with a diameter of approximately 100-500 nm, budding directly from the cell membrane in response to various stimuli (M. Yáñez-Mó, 2015, J. Extracell. Vesicles). Apoptotic bodies are products of the programmed cell death and are represented by a heterogeneous population of large vesicles with 400-3000 nm diameter, containing different components including residual organelles. Despite their different origin, exosomes, microvesicles and apoptotic bodies may harbor similar surface proteins and available isolation technologies do not allow their separation. Therefore, for these vesicles, the generic term "EV" was suggested in the community (C. Théry, 2018, J. Extracell).

Recent achievements in cancer research demonstrated, that EVs play a highly important role in diseases progression, e.g. by organ-specific preparation of the premetastatic niche (A. Hoshino, 2015, Nature). As cancer is the second most common cause of death in developed countries, cancer diagnosis and treatment became one of the most important tasks of modern medicine (S. Yusuf, 2020, Lancet). However, despite of the progress and acquired knowledge, the imminent need for new tools for early detection of cancer and new therapeutic strategies remains challenging (M. Ferrari, 2005 Nat. Rev. Cancer; L. Wu, X. Qu, 2015, Chem. Soc. Rev; E. Willms, 2018 Front. Immunol.) Recent findings show that cancer cells produce significantly more EVs than healthy cells and use EVs for the regulation of the local tumor microenvironment as well as distant sites, promote vasculogenesis, immune suppression, tumor growth and metastasis (T. B. Steinbichler, 2017, Semin. Cancer Biol.*;* J. A. Tickner, 2014, Front. Oncol.; T. M. Green, 2015, Biomed Res. Int.; I. Nazarenko, 2010, Cancer Res.) These cancer-associated EVs are promising biomarkers for early cancer detection and therapy monitoring (Y. Soung, 2017 Cancers; I. Stevic, 2020, Cells; I. Nazarenko, 2020, Tumor Liq. Biopsies; M. Nawaz, 2014, Nat. Rev. Urol.; W. Li, 2019, Adv. Mater.) as well as therapy itself (G. Nam, 2020, Adv. Mater.; P. H. L. Tran, 2020, Adv. Mater.), and can be used in proteomics (D.-S. Choi, 2015, Methods Mol. Biol. Vol. 1295 - Proteomic Profiling). However, as for their small size, high heterogeneity and the complex background of other components present in body fluids, detection and isolation of EVs still pose a considerable challenge (W. Li, 2019, Adv. Mater.; A. Clayton, 2019, J. Extracell. Vesicles). No clinically approved method for EV detection exists. In clinical use, such method should be accurate, specific, reproducible, rapid, easy to handle, cheap, and applicable for different types of EVs (S. Halvaei, 2018, Nucleic Acids).

In a first aspect, the present invention relates to a method for isolating EVs from a sample, comprising the step of contacting the sample with a supported lipid membrane (SLM) capable of binding the EVs.

As it can be taken from the examples, SLMs represent an effective tool for isolating EVs from a sample.

EVs are lipid bilayer enclosed structures, which can be secreted by almost any cell and reabsorbed by a large number of cells. They are defined by a self-contained lipid bilayer, which can additionally accommodate molecules. Non-limiting examples for EVs include exosomes (40-100 nm diameter), which are ejected by back-fusion of multi-vesicular bodies (MVBs), microvesicles (approximately 100-500 nm diameter), which are ejected directly from the membrane of a cell, and further large vesicles, so-called apoptotic bodies (400-3000 nm diameter), which can enclose various components, such as remnant organelles. According to the invention, the use of the plural in "EVs" also includes the isolation of a single EV, where appropriate.

According to the invention, a supported lipid membrane (abbreviated SLM, also referred to as "lipid patches") is characterized by a planar structure, which comprises at least two lipid monolayers, forming a lipid bilayer with a hydrophilic outer edge, which is placed on any kind of solid support. SLMs may show specific characteristics, e.g. they may retain lipid dynamics (meaning the diffusion of lipid components within the membrane) which are the basis of many interactions in natural lipid membranes, such as signaling, transport and cell-cell contacting. Furthermore, pure lipid SLMs may have intrinsic non-fouling properties (i.e. they are protein and/or cell repellant).

"Isolating" as used herein can also be understood in the sense that EVs from a sample are trapped or intercepted.

In one specific embodiment, the SLM is placed on a solid support. The combination of one or more SLMs and the solid support is also referred to as a "microarray" or "array".

According to the invention, said solid support may be any kind of solid support, e.g. a surface that can comprise different materials. Non-limiting examples for such surfaces are e.g. glass plates or plastic plates or metal plates, or combinations thereof. The surface can comprise transparent and/or opaque plates, plates with removable bottom, or plates with structured bottom (such as e.g. electrodes, and/or metal films, such as gold films and/or metal nanoparticles, such as gold nanoparticles and/or waveguides etc.). In order to produce a solid support comprising an SLM, for example lipid dip-pen nanolithography (L-DPN) can be employed, which enables patterning of micro-and nanoscale sized lipid patches on various substrates in a multiplexed manner. In one specific embodiment, the SLM is attached via physisorption to the solid support.

In a further specific embodiment, the surface opposite to the SLM comprises Polysiloxan (PS) and/or Polydimethylsiloxan (PDMS) and/or glass. Preferably, the surface opposite is coated with a protein-blocking agent. Non-limiting examples of protein-blocking agents are e.g. milk, preferably non-fat milk and/or BSA.

In a preferred embodiment, the SLM, which is attached to the solid support (i.e. the array) is placed on a microfluidic chip. A microfluidic chip is a tool, in which chemical reactions taking place can be carried out precisely within a very short time using microfluidics. This allows, for example, the integration and detection of chemical or biochemical processes on the chip.

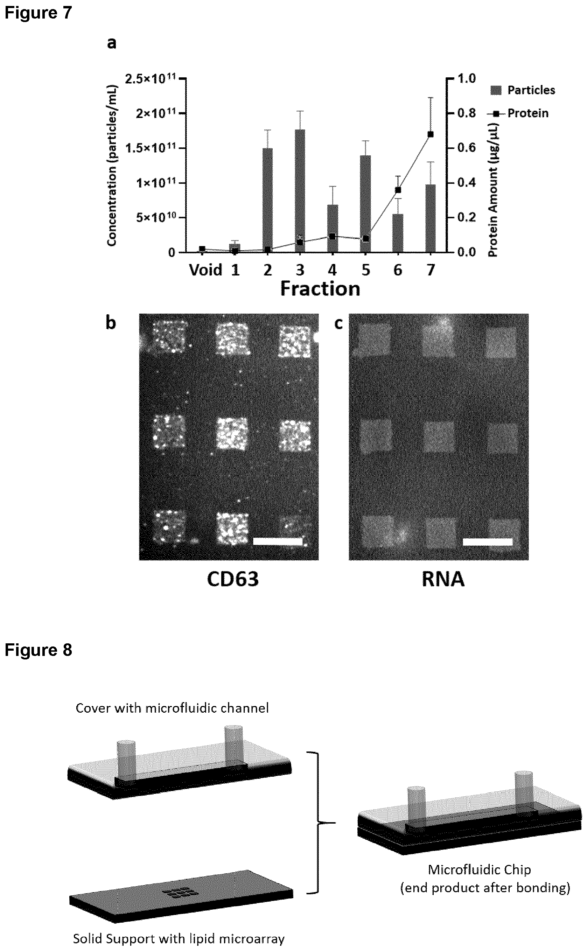

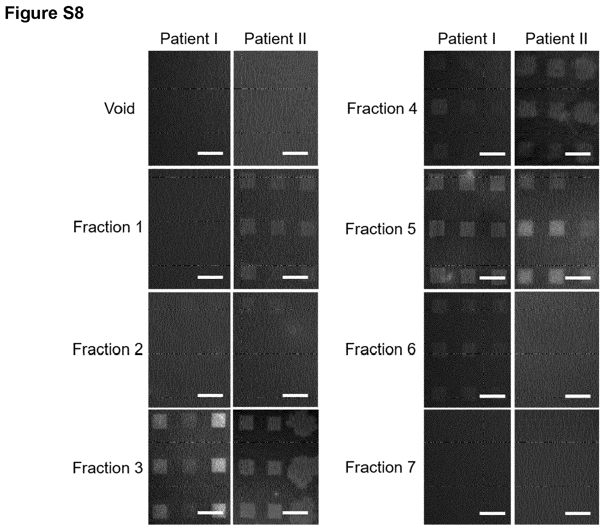

In one embodiment, a microfluidic chip comprises a microarray, consisting of lipid patches, which comprise the SLM, attached to a solid support and one or more microchannel(s) (see also Figure 8). In one embodiment, the microchannels are comprised in the cover of the microchip. Preferably, the microchannels can be used to direct the flows across the array.
In a further embodiment of the present invention, the SLM comprises structures that are anchored within the SLM. In a specific embodiment, the SLM comprises capturing molecules that are capable of binding EVs. In one particular embodiment, those capturing molecules are antibodies. These antibodies can target and bind to various surface markers of EVs. Non-limiting examples of antibodies that can be used in the procedure described herein are antibodies that can bind to: CD63, CD9, EpCAM, or any other surface molecule that is to be isolated via binding to the antibody. Thus, in a further embodiment, the antibody is directed to surface markers of a wide variety of tumor-associated EVs.

In another preferred embodiment, the antibodies are biotinylated. In another embodiment, the antibody is un-biotinylated. In still another embodiment, the antibodies are tagged. In a more preferred embodiment, the tagged antibodies are HIS-tagged. Tagging of the antibodies is not restricted to HIS-tags and also includes any other tags, such as e.g. GFP-tags, FLAG-Tags, myc-tags or any other tags that are known the skilled person in the art.

Moreover, in still a further embodiment, a biotinylated antibody can be bound to streptavidin comprised in the SLM. The natural strong binding of streptavidin to biotin is exploited herein, which allows the biotinylated antibodies to bind non-covalently to strepativin, whereby biotinylated antibodies can be anchored within the SLM. In another preferred embodiment, the capturing molecule is an un-biotinylated antibody, which may be bound to Protein A or Protein G, comprised in the SLM.

In another embodiment of the invention, the EVs are tumor-associated extracellular vesicles. Tumor-associated EVs carry selective biomarkers on their surface (surface markers) that can be isolated via recognition of those specific surface markers by corresponding capturing molecules. In particular, tumor cells secrete specific extracellular vesicles that are characteristic for them and are referred to herein as "tumor-associated extracellular vesicles". Therefore, tumor-associated EV herein relates to an EV, which is mainly produced by tumor cells and which can be characterized depending on its surface markers. Non-limiting examples for tumor-associated EV surface markers include: CD63, CD9, EpCAM, or combinations thereof.

In a further embodiment of the present invention, the sample from which the EVs are deemed to be isolated is a body fluid from a subject. In one preferred embodiment, the sample is blood from a subject. In another preferred embodiment, the body fluid is serum from a subject. In a further preferred embodiment, the body fluid can be urine from a subject.

In one embodiment, the sample is contacted with the SLM. In a preferred embodiment, the sample is passed microfluidicly across the SLM. "Passed microfluidicly" as used herein means a pressure driven flow control, wherein the sample is flown across the SLM by using capillary force driven flow. EVs that are sought to be isolated by methods related to the present invention can differ in their size. Therefore, in one preferred embodiment the EVs have a diameter of between 20 nm - 4000 nm. In a more preferred embodiment, the EVs have a diameter of between 30 nm - 3500 nm. In a most preferred embodiment, the EVs have a diameter of between 40 nm - 3000 nm.

In one embodiment of the invention, the method further comprises the step of identifying a molecule comprised in the EVs. In a specific embodiment, the molecule, which is deemed to be analyzed in a further step is a DNA molecule, an RNA molecule, a protein, a peptide and/or a lipid molecule. Also included herein are fragments or fractions of molecules like oligonucleotides or a protein portion.

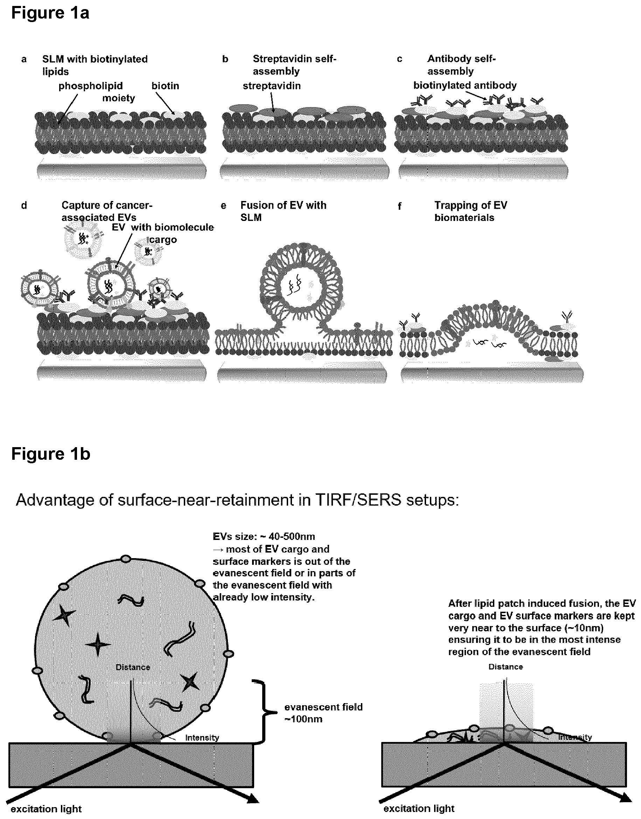

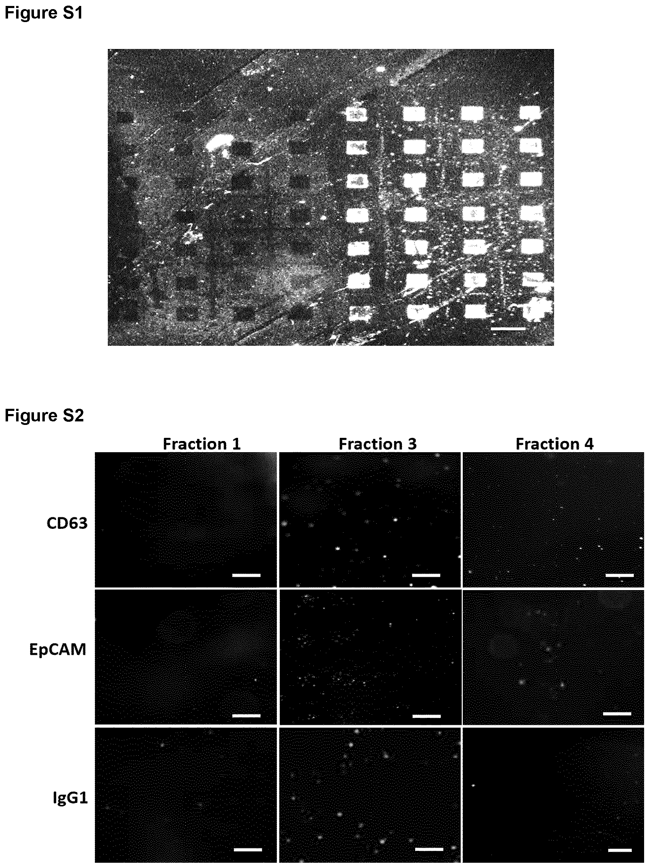

In one further embodiment, the EVs are taken up with the SLM. In this scenario, the EVs merge with the lipid bilayer of the SLM, which presses the content of the EVs to the surface to which the SLM is attached. This reduces the distance to the center of the EV, making its contents easier to examine (see also Figure 1b). Here, for example, the examination of the vesicles and their contents is facilitated for further analysis. Therefore, in one embodiment, the molecule comprised in the EVs is analyzed by staining and fluorescence detection. Non-restrictive examples for the analysis of EVs noted with the SLM include for example: Staining of the EVs and further analysis by all known techniques of fluorescence microscopy, preferably by total internal reflection fluorescence (TIRF) microscopy, or other forms of fluorescence-based techniques evaluated using a plate reader or a regular microscopy setup.

Alternatively, the molecule comprised in the EVs, which is found, after the taking up of the EVs, between the lipid bilayer and the solid support, may be further isolated , e.g. by sucking away, and then further analyzed.

In another embodiment, the EVs are not taken up with the SLM. This may imply that the EVs are stabilized, which results in the fact that the EVs do not fuse with the SLM. In this case, the vesicles together with their contents and surface markers can be captured and in a further step, for example, detached and completely removed for further analysis. The material contained in the EVs can then be further analyzed.

Irrespective of whether the EVs are taken up by the membrane or not, the EVs and / or the molecules comprised therein may be analyzed by various methods. This may include that the EVs and / or their content are further chemically analyzed by spectroscopy. In a more preferred embodiment, the spectroscopy method is SERS (surface-enhanced raman spectroscopy). In another preferred embodiment, the spectroscopy method is TIRF (total internal reflection fluorescence spectroscopy). In even another preferred embodiment, the spectroscopy method is ATR spectroscopy (attenuated total reflection spectroscopy).

Due to the small distances, the use of SERS for the analysis of the molecule comprised in the EVs is also envisaged in cases where the EVs are taken up by the SLM, but the content is not further isolated.

Non-limiting examples of methods by which said molecule can be analyzed after further isolation include, for example, PCR (polymerase chain reaction), HPLC (high performance liquid chromatography) or any other form of chromatography, mass spectrometry (such as MALDI-MS (matrix assisted laser desorption/ionization mass spectrometry) or ESI-MS (electrospray ionization mass spectrometry)) for downstream genomics and/or proteomics.

In a specific set-up, in which the EVs are removed from the SLM again, morphologically intact EVs can also be prepared for analysis via electron microscopy. For this purpose, the EVs can be collected using micro-capillaries sampling, for example.

In a second aspect of the invention, a method for diagnosing a tumor is provided, comprising the steps of i) performing the method according to the first aspect of the present invention and ii) identifying a molecule comprised in the EVs as a tumor molecule according to the methods of the first aspect, thereby diagnosing the tumor.

A "tumor molecule" as it is used herein is defined as any molecule that is comprised in an EV, which is derived from a tumor cell.

Considering the fact that the method for isolating EVs underlying the invention does not have to be considered tumor-specifically, a variety of non-limited tumor types can be diagnosed using this method. Tumors to be diagnosed by the method of the present invention can either be benign or malign. Examples of tumor types include, but are not limited to those: pancreatic cancers, brain tumors, bladder cancer, breast cancer, colon and small bowel cancer, ovarian cancer, gallbladder cancer, liver cancer, uterine cancer, stomach cancer, laryngeal cancer, cancers of the skin (white and black melanomas), testicular cancer, lymphoma, leukemia, bone cancer, lung cancer, myeloma, oral cavity cancer, prostate cancer, thyroid cancer, esophageal cancer, or soft tissue cancer.

A third aspect of the present invention relates to a microfluidic chip comprising a SLM for isolating EVs, which is characterized by the features according to the first aspect of the present invention. In one preferred embodiment, the SLM is attached via physisorption on the microfluidic chip. In another preferred embodiment, the surface opposite to the SLM comprises Polysiloxan (PS) and/or Polydimethylsiloxan (PDMS) and/or glass. Preferably, the surface opposite is coated with a protein-blocking agent. Non-limiting examples of protein-blocking agents are e.g. milk, preferably non-fat milk and/or BSA.

A fourth aspect of the invention relates to the use of a microfluidic chip for isolating EVs by using the methods disclosed in the context of the invention.

The invention is further explained by the attached figures and examples, which are intended to illustrate, but not to limit the present invention.

### DESCRIPTION OF THE FIGURES

**Figure 1a****:** Scheme of extracellular vesicle (EV) capture by supporting lipid membranes (SLM). a) Fabrication of biotinylated SLM arrays by L-DPN. b) Coating of the SLM arrays with streptavidin. c) Binding of biotinylated antibodies on the SLM arrays. d) Capture of tumor-associated EVs on SLM arrays. e) Fusion of captured EVs with the SLM. f) Trapping of EV-derived biomaterials (e.g. RNA, proteins) by the SLM.
**Figure 1b****:** Illustration of the Advantages of surface-near-retainment in TIRF/SERS setups: After lipid patch induced fusion, the EV cargo and EV surface markers are kept very near to the surface (∼10nm) ensuring it to be in the most intense region of the evanescent field.

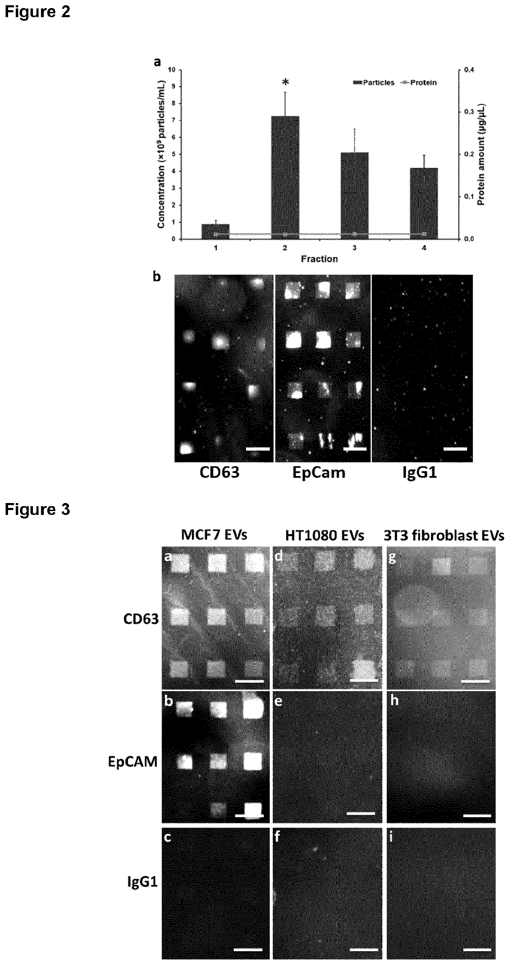

**Figure 2****:** Capture of purified MCF7 EVs. a) Total protein amount (orange line) and concentrations of particles (blue bars) of fraction 1 to 4. Fraction 2 (marked with *****) was chosen for subsequent experiments as it contains the highest number of particles and low protein concentration, confirming EV enrichment in these fractions according to the manufacturer recommendations. Error bars are given as standard error of the mean in 4 independent samples. b) Consistent with the expectations, fraction 2 also leads to the strongest fluorescent signals (compared to the other fractions) on lipid patches harboring CD63 and EpCAM antibodies. Patches functionalized with IgG1 as isotype control show no fluorescent signal after incubation with fraction 2 (negative control). Scale bars equal 50 µm.

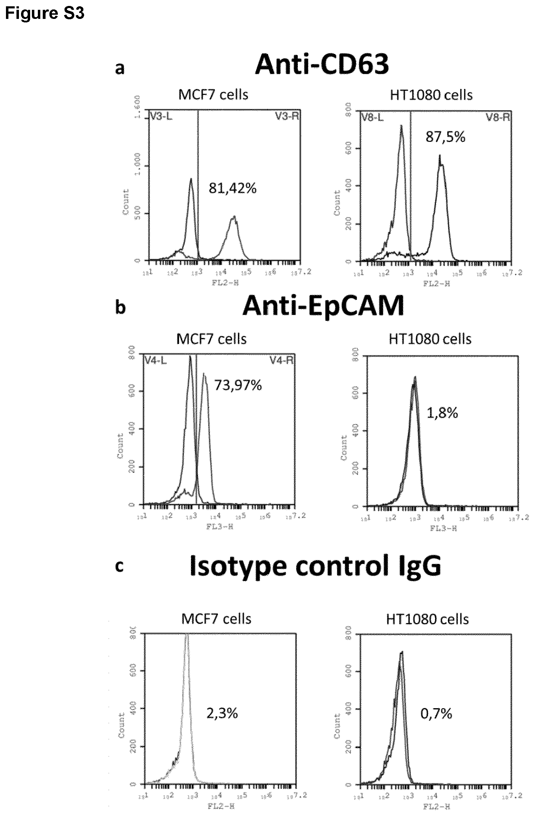

**Figure 3****:** Specificity of EV capture. Fluorescently labeled (red, PKH26 dye) MCF7-derived EVs are captured by a) CD63 and b) EpCAM antibody-functionalized lipid patches. c) No capture is observed on lipid patches functionalized with IgG1 isotype control antibodies (negative control). Similarly, fluorescently labeled EVs (green, PKH67 dye) derived from HT1080 cells are captured by d) CD63 antibody-functionalized lipid patches. e) A minor signal of HT1080 EVs on the EpCAM-functionalized lipid patches was detected, indicating that high EV concentration may cause some unspecific capture. f) Again, no interaction with IgG1 isotype control antibodies carrying lipid patches is observed (negative control). Fluorescently labelled (green, PKH67 dye) 3T3 fibroblast derived EVs are only captured on g) CD63 antibody-functionalized lipid patches but not on h) EpCAM antibody-functionalized lipid-patches, or i) IgG1 isotype control antibody-carrying lipid patches (negative control). Scale bars equal 50 µm.

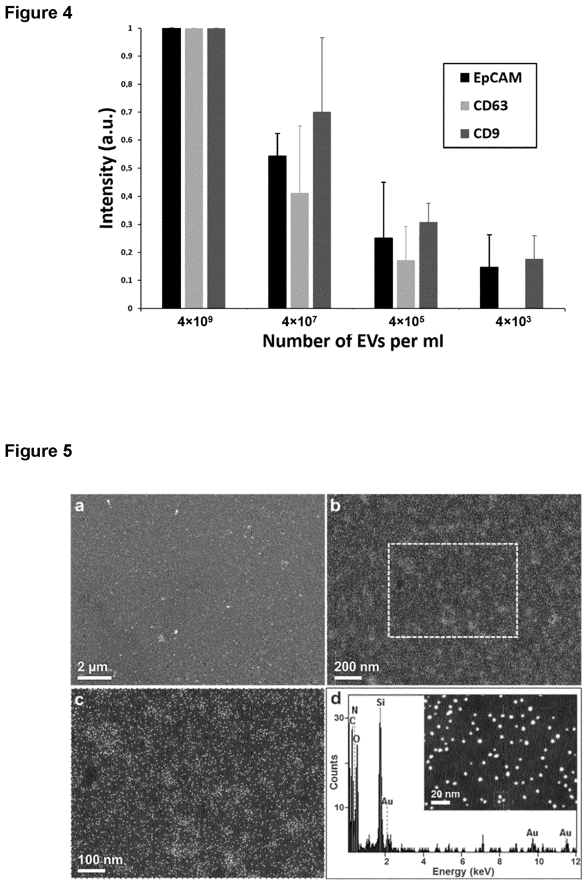

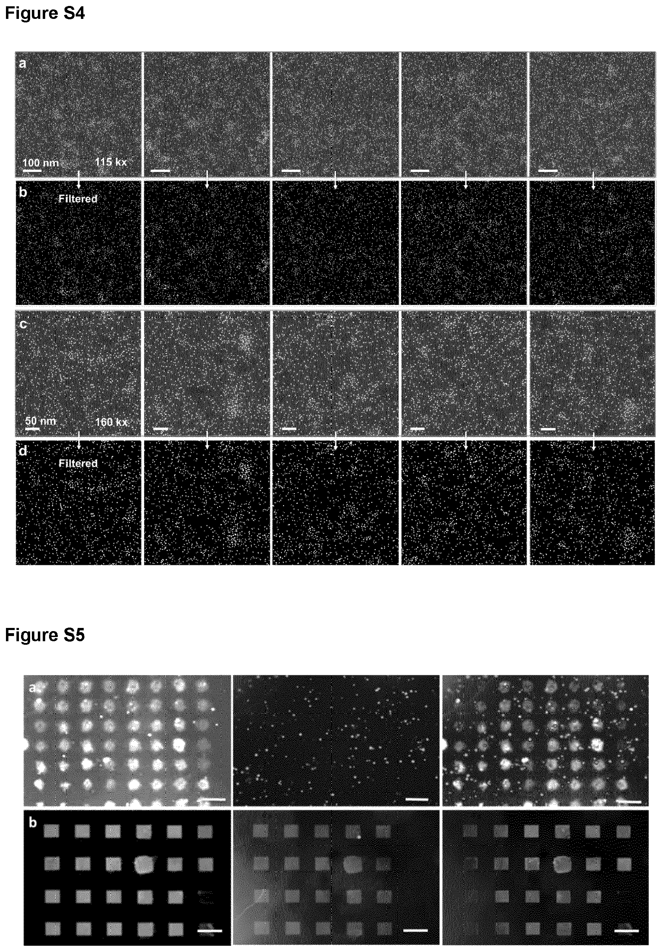

**Figure 4****:** EV capture sensitivity. The graph shows the obtained fluorescence intensity on the lipid patch array functionalized with different antibodies (CD9, CD63, and EpCAM, respectively) for undiluted purified MCF7-derived EVs (containing 4x109 EVs per mL) and dilutions of 1:102, 1:104, and 1:106, containing 4x107, 4×105, 4×103 EVs per mL, respectively. The fluorescence intensity obtained with undiluted EVs is set to 1 for normalization. The error bars represent the SD from biological triplicates for each antibody.
**Figure 5****:** Antibody density on functionalized lipid patches. a), b) STEM images of an antibody-functionalized lipid patch after incubation with Au-NP conjugated secondary antibody. c) close-up of the area marked with the green box in (b). At this magnification, single Au-NPs become discernable. d) EDX data on the particles within the red box of the inset, showing that the observed particles are indeed the Au-NPs. Based on the observed Au-NP density, 5.1 × 106 antibodies are expected per 30 × 30 µm² lipid patch (translating to a density of ∼5.7 × 103 µm-1).

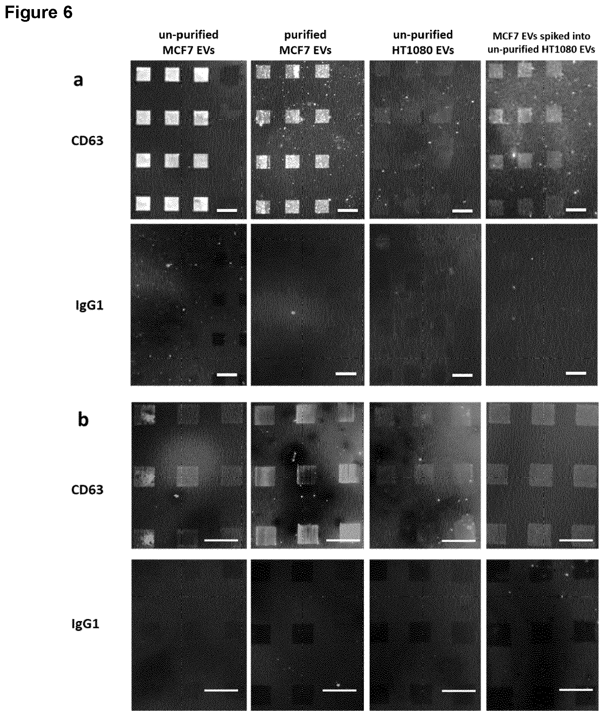

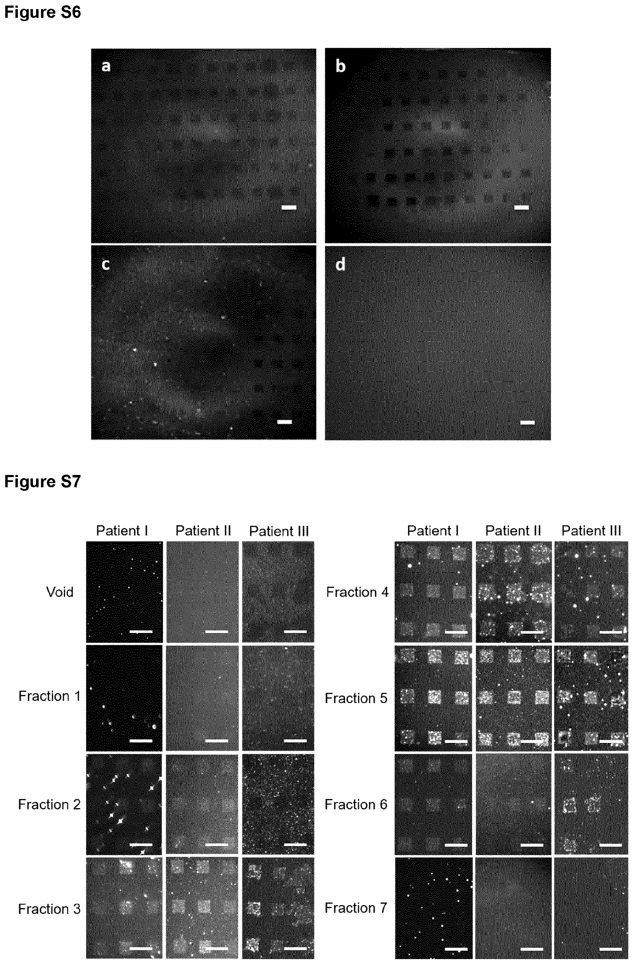

**Figure 6****:** EV capture from complex samples and RNA retention. a) Fluorescence microscopy images of CD63 antibody- and IgG1 (isotype control) antibody-functionalized lipid patches after capture of EVs from purified, un-purified and spiked samples and subsequent immunostaining against EpCAM (anti-EpCAM antibody from rabbit, Alexa-647 (in purple) conjugated anti-rabbit antibody). MCF7-derived EVs result in a strong fluorescence signal from purified and un-purified sample. Incubation of unpurified HT1080 derived EVs results (as expected) in only weak fluorescence. Incubation of HT1080 conditioning medium spiked with purified MCF7-derived EVs also induces a fluorescence signal on the lipid patch array. The IgG1 (isotype control) carrying lipid patches remain without fluorescence for all cases (negative control). This shows that specific detection out of complex medium is possible, and the signal arises not from unspecific interactions with other components of the sample medium. b) Staining with a nucleic acid dye (SYTO in green) reveals that all CD63 antibody-functionalized lipid arrays contain nucleic acids, while arrays carrying IgG1 (isotype control) antibodies do not. This shows, that RNA cargo present in the EVs remains in the lipid patch arrays after capture, thus could be used for downstream analysis. All scale bars equal 50 µm.
**Figure 7****:** EV capture from patient samples. a) NTA/BCA data on the different fractions obtained from 3 cancer patients. The error bars report the SD. b) Fluorescence microscopy images of CD63 antibody-functionalized lipid patches after capture of EVs from fraction 5 of patient I and subsequent immunostaining against EpCAM (anti-EpCAM antibody from rabbit, Alexa-647 (in purple) conjugated anti-rabbit antibody) and c) staining for nucleic acid (SYTO in green) confirming RNA cargo retention. Scale bars equal 50 µm.
**Figure 8****:** Schematic structure of the microfluidic chip. A cover comprising microchannels is placed on top of a solid support comprising a lipid microarray, comprising an SLM for isolating EVs.
**Figure S1:** Control experiment for unspecific PKH dye adhesion. Fluorescent microscopy image of a lipid microarray after incubation with PKH26 stained MCF7 EVs. Only the EpCAM antibody-functionalized array columns on the right light up in fluorescence, while the non-functionalized (DOPC only) columns on the left remain dark. This indicates that no unspecific adhesion of PKH dye or unspecific EV fusion with non-functionalized lipid patches occurs. The non-functionalized patches even appear darker compared to the substrate background, indicating the non-fouling properties of DOPC which even suppresses unspecific adhesion compared to the naked substrate. Scale bar equals 50 µm.
**Figure S2:** Control experiment with other purification fractions. Incubation of the other (non-EV) fractions of the purification process on AB functionalized lipid microarrays does not lead to any significant fluorescence signals from the lipid patches. Scale bars equal 50 µm.
**Figure S3:** FACS analysis of MCF7 and HT1080 cells. a) Both cell lines are CD63 positive. b) Only MCF7 cells are strongly EpCAM positive with only minor interaction in HT1080. c) Both cell lines show negligible interaction with the isotype control IgG.
**Figure S4:** STEM images of Au-NPs bound to a lipid patch. a) The original STEM images (magnification of 115.000x, scale bars equal 100 nm) of the 2 nm sized Au-NPs on the lipid patch. b) Pictures after identification of Au-NPs by ImageJ. The area of each image is 666 × 666 nm² or 0.443 µm². The average number of Au-NPs in each image is 2530 ±101. Extrapolating from these images, around 5.712 × 10³ ± 228 Au-NPs are bound per µm². This translates to ∼5.1 × 10⁶ ± 0.2 × 10⁶ ABs on a lipid patch of 30 × 30 µm². As consistency check, the analysis was repeated with c) even higher magnification images (160.000x, scale bars equal 50 nm), d) thresholded in the same way. Here, 1269 ± 35 particles are counted per area of 471 × 471 nm², yielding 5.723 × 10³ ± 159 Au-NPs bound per µm². Extrapolating onto a whole patch, ∼5.1 × 10⁶ ± 0.2 × 10⁶ ABs are expected, in agreement with the analysis of the lower magnification images.
**Figure S5:** Direct surface immobilization vs. lipid patch based immobilization. a) Fluorescent image of a click-chemistry bound CD63 AB microarray after incubation with fluorescently labelled MCF7 EVs (PKH67 dye, green fluorescent). To visualize also the microarray itself, fluorescently labelled streptavidin (streptavidin-Cy3, red fluorescent) was used for building up the AB sandwich structure. While the microarray it-self is clearly visible (left), only random attachment of EVs is observed (middle) with no correlation between array features and EV attachment visible in the overlay (right). b) The parallel experiment on the lipid microarray bound ABs shows a perfect correlation between the lipid microarray (left), captured EVs (middle) in the overlay image (right). Scale bars equal 50 µm.
**Figure S6:** Control experiments for EV capture from unpurified conditional medium. a) To exclude unspecific binding of EVs or other interfering components of the conditional medium to the lipid microarrays were coated only with streptavidin, not carrying any anti-CD63 AB. After incubation with MCF7 EVs from unpurified conditional medium and subsequent staining against EpCAM (rabbit anti-EpCAM AB, Alexa-647 conjugated anti-rabbit secondary AB), no signal rises on the lipid patches, indicating a successful negative control (no unspecific binding). b) To exclude unspecific interactions of the secondary detection AB, lipid microarrays with anti-CD63 AB were incubated with MCF7 EVs as before, but then directly incubated with the secondary detection AB (Alexa-647 conjugated anti-rabbit AB) without prior incubation with the primary detection AB (rabbit anti-EpCAM AB). No signal is visible on the lipid patches, showing that the secondary detection AB has no significant unspecific interaction with the lipid microarrays and EVs. c) Lipid microarrays carrying IgG1 isotype control AB show no detection signal after MCF7 EV incubation and subsequent staining, indicating no unspecific interaction of EVs and the lipid microarrays. d) When anti-CD63 AB carrying lipid microarrays, after incubation with MCF7 EVs, are incubated with rabbit IgG isotype control AB (instead of rabbit anti-EpCAM detection AB), subsequent incubation with the fluorescently labelled anti-rabbit secondary AB also induced no signal. This indicates that there is also no unspecific interaction between non-target detection ABs and the lipid microarrays or the bound EVs. The scale bars in all images equal 50 µm.
**Figure S7:** EV capture from patient samples. The image shows CD63 antibody-functionalized lipid patches after EV capture from patient sera and staining against EpCAM (rabbit anti-EpCAM AB, Alexa-647 conjugated anti-rabbit secondary AB). The columns correspond to the different patients, the lines then give the results for different fractions and the negative control (Void). All scale bars equal 50 µm.
**Figure S8:** RNA retention from patient samples. For two of the patients, additional staining for RNA detection was performed. The image shows CD63 antibody-functionalized lipid patches after EV capture from patient sera and staining for nucleic acid with SYTO confirming RNA cargo retention. The columns correspond to the different patients, the lines then give the results for different fractions and the negative control (Void). All scale bars equal 50 µm.

### EXAMPLES

### Example 1: The lipid microarray based EV capture platform

In order to produce SLM microarrays, lipid dip-pen nanolithography (L-DPN) was employed, which enables patterning of micro- and nanoscale sized lipid patches on various substrates in a multiplexed manner (i.e. different compositions of lipid within one array) (J. van Weerd, 2015, Adv. Healthc. Mater; M. Hirtz, 2011, Langmuir; M. Hirtz, 2013, Nat. Commun.; A. Urtizberea, 2015, Nanoscale*).* First, lipid membranes of 1,2-dioleoyl-sn-glycero-3-phosphocholine (DOPC) with a 5 mol% admixing of the biotinylated phospholipid 1,2-dioleoyl-sn-glycero-3-phosphoethanolamine-N-(cap biotinyl) (Biotin-PE) are written via L-DPN (Figure 1a(a)). Then these are incubated with streptavidin solution to provide binding sites for biotinylated antibodies (ABs) (Figure 1a(b)). Specific biotinylated ABs recognizing tumor-associated EVs are incubated with the array and self-assemble onto the lipid patches (Figure 1a(c)). The ABs can also be delivered to individual patches by spotting techniques, in case multiplexed detection arrays are desired (H. Y. Liu, 2020, Molecules). When the arrays are exposed to liquids containing EVs carrying the targeted surface markers, these will attach to the lipid patches carrying matching ABs (Figure 1a(d)). In addition to the mere attachment, the SLM based approach allows for membrane fusion taking place between captured EVs and the lipid patches in the array (Figure 1a(e)). In natural membrane fusion, van der Waals attraction and/or membrane proteins bring two lipid membranes close together leading to increased membrane disorder, defects occurring in the bilayers then lead to final fusion (G. Ce, 1999, Adv. Drug Deliv. Rev; L. V. Chernomordik, 2008, Nat. Struct. Mol. Biol.)*.* Albeit the molecular mechanisms of EV uptake by the target cells are not fully discovered, one of the possible mechanisms, suggested is fusion. To allow the EV to fuse with the cell membrane, an EV should be kept in a close proximity to the membrane of the recipient cell. It is suggested, that SNARE and Syncytin-2 may be involved in the membrane fusion (S. Martens, 2008, Nat. Rev. Mol. Cell Biol.; J. Han, 2017, Front. Physiol.; I. Prada, 2016, Int. J. Mol. Sci.; L. A. Mulcahy, 2014, J. Extracell. Vesicles); nonetheless, it is assumed, that contact of EV with the cell membrane may be sufficient to initiate fusion (P. Zhou, 2013, Neuron; B. van Lengerich, 2013, Biophys. J). In the platform used herein, the membrane bound ABs, therefore, are not only ensuring specificity of EV capture, but also promote membrane fusion and enhance the trapping processes, by holding the EV in near vicinity to the lipid patch. Finally, the membrane fusion process leads to trapping of EV cargo into the membrane patch (Figure 1a(f)), which may open the route to further downstream analysis of the cancer-related genome and proteome. The platform is free in choice of ABs and thus very flexible in the desired target. ABs can be chosen from a library of available options to address specific needs in regard to the targeted EVs. Several tetraspanins, in particular CD9, CD63, and CD81, were found to be enriched on the majority of EVs and thus are frequently used as generic EV biomarker. In many tumor-associated EVs, the epithelial cell adhesion molecule (EpCAM) was identified. Therefore, CD63 and EpCAM were selected herein as general and as tumor-specific EV biomarker respectively.

Phospholipids: 20 mg/mL (25.4 mM) of 1,2-dioleoyl-sn-glycero-3-phosphocholine (DOPC; Avanti Polar Lipids, U.S.A.) and 5 mol% of 1,2-dioleoyl-sn-glycero-3-phosphoethanolamine-N-(cap biotinyl) (Biotin-PE; Avanti Polar Lipids, U.S.A.) in DOPC were used in patterning the lipid patches. All lipids were purchased from Avanti Polar Lipids, U.S.A.

Patterning of lipid arrays by DPN: The lipid micropattern patches were fabricated by DPN 5000 (Nanoink Inc., U.S.A.) with the B-side of M-type cantilever array which has 12 tips with a 66 µm pitch (Advanced Creative Solutions Technology (ACST, U.S.A.). Tips were homogenously coated by dipping into the matched inkwells (ACST, U.S.A.) for 5 minutes with a controlled 60 % relative humidity (RH). The inkwell was loaded with 2 µL of the desired phospholipid mixture (20 mg/mL in chloroform) in each reservoir and chloroform evaporated in a vacuum desiccator (15 min) or overnight in a damp-proof box. Afterwards, the coated tips were moved to the desired location on the substrate (either glass cover slips cleaned by sonification subsequently with chloroform, isopropanol and DI water, or 2-methacryloyloxyethyl phosphorylcholine (MPC) copolymer covered slides and lipid patch arrays were written. The patches (30 µm × 30 µm) were written at 30% - 40% RH with hatch lines of 0.5 µm pitch and a writing speed of 0.2 to 2 µm/s.

EV chip functionalization and capturing: The micropattern lipid patches on EV chip were coated by 1% streptavidin (Sigma-Aldrich, U.S.A.) in 10% BSA (Sigma-Aldrich, U.S.A.) in PBS for 30 minutes and washed 3 times by pipetting on and off 100 µL of PBS to wash away unbound streptavidin. The streptavidin-lipid patches were then incubated with 100 µl of 0.01 µg/µL solution of desired biotinylated antibody ((Biotinylated anti-EpCAM (VU-1D9, abcam, U.K.), biotinylated anti-CD63 (MEM-259, abcam, U.K.), biotinylated anti-CD63 (Rabbit anti-mouse; MyBioSource, U.S.A.) and biotinylated Mouse-IgG1 isotype control (MOPC-21, abcam, U.K.)) in 10% BSA in PBS for 30 minutes and washed 3 times by pipetting on and off 100 µL of PBS, rendering the EV chip ready to use. The purified EVs were diluted to 102-108 times with 4% EDTA-free Protease Inhibitor cocktail (Sigma-Aldrich, U.S.A.) in cold PBS. The purified and diluted EVs fraction (50 µL) was loaded onto the prepared EV chip for 1 hour at 4 °C. After incubation, the sample was washed by dipping it into 100 mL of DI water for 3 times. Samples where then imaged with a fluorescence microscope.

### Example 2: Capture of tumor-associated EVs by SLM arrays

To establish a proof-of-concept for the platform, EVs from the conditional medium of the broadly used breast cancer line MCF7 were utilized. To obtain standardized material for experimentation, small EVs were purified using size exclusion chromatography (SEC) and characterized according to the MISEV guidelines (C. Théry, 2018, J. Extracell. Vesicles). The fractions 1 to 4 obtained from SEC were collected to determine the EV-enriched fraction. For that, the protein content and number of particles were measured by micro bicinchoninic acid assay (BCA) and nanoparticle tracking analysis (NTA), respectively (Figure 2a). Following recommendations of manufacturer we selected fraction 2, exhibiting the highest number of particles and (as all fractions 1-4) a low amount of protein. In order to visualize the EVs in fluorescence microscopy, they were stained with a lipophilic dye (PKH26, red fluorescent), and incubated to lipid microarrays functionalized with CD63 and EpCAM ABs (Figure 2b). The lipid patches of the array exhibit red fluorescence in the presence of EVs bound either to CD63 or EpCAM, while lipid patches carrying isotype control ABs (immunoglobulin G (IgG)1) remain dark (Figure 2b). This shows that the lipid array can capture EVs by specific surface biomarkers (CD63 or EpCAM), while no fusion occurs when no matching biomarker is present on the EV (as seen on the sample containing the IgG1 isotype control). As the used dye is lipophilic, an additional control experiment was performed to ensure that the positive fluorescence signals on the lipid array are not caused by PKH dye not bound to EVs or detaching from the EVs and diffusing towards the array. For this, purified and stained EVs were incubated on a lipid array consisting of patches with pure DOPC on one side as negative control and Biotin-PE/DOPC and AB functionalized patches on the other side. Here, as expected, only the AB-functionalized patches emit the fluorescence signal, while the DOPC-only patches remain dark (Figure S1). This observation supports the specificity of the visualized fluorescence signal, indicating a binding of labeled EVs to the AB-functionalized patches. Furthermore, this experiment highlights the anti-fouling property of the lipid layer, as the DOPC patches appear even darker then the surrounding non-functionalized glass-slide surface, on which unspecific random adhesion of some of the stained EVs may take place. As an additional control, other SEC fractions were incubated on AB-functionalized lipid microarrays. As expected, no significant fluorescence signal is observed on any of the patches functionalized with CD63, EpCAM ABs, or the IgG1 isotype control, supporting on the one hand the quality of EV isolation and on the other hand the specificity of the SLM-based EV detection (Figure S2). In combination, these results show that the lipid microarray platform can effectively capture EVs purified from the conditional medium of MCF7 cells as a proof-of-concept.

Vesicles labeling with PKH dyes: After the ultrafilter, Diluent C buffer (Sigma-Aldrich, U.S.A.) was added into condensed medium (total volume 1 mL) and then mixed well with 1 mL of labeling solution (consisting of 6 µL of the PKH67 or PKH26 ethanolic dye (as obtained from Sigma-Aldrich, U.S.A.) in 1 mL Diluent C). The mixture was incubated for 5 min at room temperature and to stop the staining reaction, 1% BSA (Sigma-Aldrich, U.S.A.) in PBS buffer (2 mL) was added for 1 min.

Micro BCA analysis: 150 µL of each standard and fractions were loaded into a microplate's well and replicated twice. Each well was added with 150 µL of the working reagent and mixed thoroughly on a plate shaker for 30 seconds. The loaded microplate was then incubated for 2 hours at 37 °C. The result was measured for absorbance at 562nm on a plate reader.

Nanoparticle Tracking Analysis (NTA): EV concentration and size distribution were determined by ZetaView (Particle Metrix, Germany). Samples were diluted consecutively with ddH20 to receive an optimal concentration of 100-200 particle counts. One mL of diluted sample was injected into the NTA. The size distribution was measured in scatter mode and Zeta potential was measured in Zeta Potential mode using set up: Sensitivity 85; Min size 20 nm; Max size 1000 nm; Min Brightness 10. The ZetaView determines the size of vesicles based on Brownian motion and this principle is used for analysis of nanometer-sized particles.

### Example 3: Specificity of EV capture

Clinical samples, such as blood samples, contain various EVs originating from different cell types, therefore, specific detection of tumor-derived EVs is crucial for a robust diagnosis. To explore the specificity of our device, purified EVs from three different cell lines (MCF7, HT1080 and 3T3 fibroblast) were trialed. As a general EV biomarker, CD63 should be present on EVs released by any of the three cell lines. In contrast, only MCF7 (a breast cancer cell line of epithelial origin), but not HT1080 (a non-epithelial fibrosarcoma cell line) express EpCAM, as confirmed by fluorescence-activated cell sorting (FACS) (Figure S3). When incubating the respective EVs on AB-functionalized lipid microarrays, the fluorescence signals reveal the described specificity (Figure 3). All three types of EVs are captured on the CD63 functionalized lipid patches. In contrast, only the MCF7-derived EVs are captured on EpCAM-functionalized lipid patches. For the IgG1 isotype control AB none of the EV types is captured (as expected from the negative control). The results show, that by choice of adequate AB, the platform can specifically capture a certain type of EV.

### Example 4: Sensitivity of EV capture

In order to investigate the sensitivity of our platform, EV dilution series were conducted for lipid microarrays functionalized with three different ABs (against CD9, CD63, and EpCAM, respectively). The concentration of MCF7-derived EVs in nine purified samples from independently collected cell culture medium was quantified by ExoELISA to be (3.7 ± 2.2) × 1010 particles/mL. The initial sample was diluted by a factor of ten to obtain a starting point with ∼4 × 109 EVs/mL for a dilution series in the physiological relevant range of EV abundance (103-109/mL) in patient blood. This sample range (4 × 109 EVs/mL to 4 × 103 EVs/mL) was then incubated onto lipid arrays functionalized with different capture ABs. The obtained fluorescence signals show that detection is still possible down to 1: 104 dilution (translating to 5 × 105 EVs/mL) for CD63 ABs and even in a 1:106 dilution (translating to 5 × 103 EVs/mL) for the EpCAM and CD9 ABs (Figure 4), thus demonstrating the high sensitivity in the relevant EV concentration range. Additionally, the differences between the three ABs underline, that choice of an optimal AB is key for an efficient capture and maximal sensitivity.

EV dilution series: For the dilution series experiments, MCF7 EVs were purified as described above and EV concentration was determined by ExoELISA. Then the EV samples were diluted to the respective concentration and incubated on lipid patch arrays functionalized with the respective ABs as described above. After EV capture, the samples were stained and analyzed by fluorescence microscopy. Fluorescence intensities for 5 patches on each array were quantified with the onboard software of the microscope (NIS-Elements, Nikon, Germany). For each AB the experiment was done in triplicate with independently prepared EV samples.

ExoELISA: The ExoELISA-Ultra CD63 kit (System Biosciences, U.S.A.) was used for calculating EV concentration for the purified samples used in the dilution series. 50 µL of EV samples and standards were loaded into microtiter plate and incubated at 37 °C for 1 hour. After incubation, the reacted wells were washed 3 times by 100 µL of 1X wash buffer for each well. 50 µL of diluted CD63 primary antibody (1:100) in blocking buffer was added to each reacted well and incubated at room temperature for 1 hour with shaking (parameter). Before adding 50 µL of 3,3',5,5'-tetramethylbenzidine (TMB) substrate to each reacted well, washing step with 1X wash buffer was repeated. After incubating with TMB substrate at room temperature for 5-15 minutes with shaking, 50 µL of stop buffer was loaded to each well and read immediately by an ELISA reader at 450 nm wavelength.

### Example 5: Characterization of AB immobilization

To further characterize the platform, the immobilization density and role of the lipid membrane fluidity in the capture process was investigated. In order to investigate the average amount of immobilized ABs on a lipid patch, we used gold nanoparticle (Au-NP)-conjugated secondary ABs to detect the EV-capture ABs bound to the lipid patches. The Au-NPs were then visualized on the lipid patches by scanning transmission electron microscopy (STEM). The 2 nm Au-NPs are clearly visible in the obtained images (Figure 5). As counting the particles on a whole 30 × 30 µm² lipid patch is not feasible, 10 smaller areas in two different magnifications and on different locations on a patch were scanned and the particles were identified and counted using ImageJ (Figure S4). The size of one AB is around 10 nm, thus the Au-NPs (2 nm) are significantly smaller and should not sterically affect the binding density. Although we cannot fully exclude the possibility of double conjugation, it seems reasonable to assume that there is a 1:1 ratio of primary AB to secondary AB and conjugated Au-NP (or being at least of the same order). Following these assumptions, an average number of around 5.1 × 106 ABs per 30 × 30 µm2 lipid patch, giving a density of 5.7 × 103 µm-1. This is about a tenth of the number of accessible headgroups of a model allergen conjugated lipid in a lipid patch as estimated previously (S. Sekula-Neuner, 2012, Small) when adjusted to the same concentration. Keeping in mind that each streptavidin (as tetramer with a total of four biotin-binding pockets, 2 each in opposite directions) can potentially bind to biotinylated lipids at the membrane facing side, but only one of the bigger biotinylated ABs on the other side for steric reasons and that conjugation efficiency of the AB attachment to the lipid patch and the secondary conjugation with the Au-NP carrying AB will both be not 100%, these results are still in reasonable agreement. In addition to the previously discussed AB density, the role of attachment to the lipid layer in capture effectiveness is of interest. Various strategies for AB immobilization are discussed in the literature, in particular focusing on how to minimize the impact of linking strategy on AB function. While common chemical linking approaches, targeting amine-, carboxyl- or thiol- groups usually work effortless, they cannot control the specific orientation of AB in relation to the surface, as these groups are available in abundance on the AB. Therefore, a fraction of the ABs will be oriented with their active site towards the surface, thus rendering them inactive for capture. Certain click-chemistry approaches as well as making use of biochemical interactions (e.g. streptavidin-biotin, protein A/G binding to ABs) can achieve an orientation-controlled immobilization. However, these approaches are usually more elaborate, and e.g. in the streptavidin-biotin strategy, also the ABs need to be biotinylated site-specifically, otherwise orientation is again random. All standard approaches have in common, that they bring the AB usually into very near vicinity to the surface with only a linker molecule in between. Therefore, it is of special interest, that it is reported in the literature, that anchoring of ABs to lipid membranes enhances the capture of circulating tumor cells (CTC) and that tuning membrane fluidity of liposomes can be used to specifically target cancer cells. This points to a positive influence of such a "lipid cushion" on AB effectiveness in general. In contrast, commercial EV capture platforms like ExoView (Nanoview Biosciences, USA) and Exo-Check^{™} (System Biosciences, USA) and approaches using plasmonic sensors directly immobilize antibodies on their devices. Therefore, an approach for the case of ABs directly bound to the solid substrate was trialed, leaving out the lipid patch cushion. To bind the ABs directly to a substrate, a click-chemistry approach was used to generate surface bound biotin arrays as described previously. In short, biotin-azide was spotted via microchannel cantilever spotting (µCS) onto dibenzocyclooctyne (DBCO) functionalized glass slides. Then biotinylated ABs were bound via streptavidin as linker resulting in arrays analogue to the ones described above, but without the lipid patches in between bound ABs and glass surface. This allows for a side-by-side comparison of directly substrate-bound ABs (with only a short and rather rigid linker molecule between the glass and streptavidin/AB part of the sandwich) and the lipid patch based AB microarrays (with a full lipid membrane in fluid state between the streptavidin/AB and the supporting solid glass substrate). After incubating CD63 AB-functionalized arrays of both types with fluorescently labelled EVs, the correlated fluorescence signal can only be detected on the lipid-based microarrays, while the direct substrate-bound sample only shows random attachment of EVs (Figure S5). This striking difference can be accounted for by two reasons: (I) the mobility of lipid-linked ABs within a lipid patch, and (II) denaturation of ABs in direct contact to the solid substrate. Orientation significantly affects the activity and efficiency of ABs and catalytic proteins. Here, the lipid-membrane introduces a greater flexibility to the attachment, due to the membrane fluidity, potentially mitigating accessibility problems. A similar effect was also reported in the capture of CTCs by lipid-conjugated ABs, where the capture efficiency decreased significantly (from 82.1% to 63.5%) after fluidity of the utilized SLMs was reduced. Furthermore, the direct contact of the ABs with the solid surface can lead to denaturation and loss of function. The non-fouling properties of the lipid patches can minimize the impact on AB structure, as they interact much less strongly with the bound AB as a glass surface.

Electron Microscopy: A FEI Titan 80-300 aberration (image) corrected transmission electron microscope (TEM) operating at 300 KV acceleration voltage and equipped with a high-angle annular dark-field (HAADF) detector (Fischione) for Scanning TEM (STEM) imaging, and a S-UTW EDX detector (EDAX Inc.). TEM samples were prepared by direct printing. The lipid micropattern patches were directly fabricated on a silicon nitride (SiN) 20 nm thick membrane TEM window grid with 9 electron-transparent windows, 8 of dimensions 100 × 100 µm², and one of 100 × 350 µm², by writing a lipid mixture of DOPC and Biotin-Cap-PE with a DPN 5000 system (Nanoink Inc., U.S.A.). The details of printing condition can be seen above section (Patterning of lipid arrays by DPN). The patterns on the SiN grid were coated 1% streptavidin and then incubated with 0.01 µg/µL of primary biotinylated-antibody from rabbit (Abcam, U.K.). After washing, the primary antibody-lipid arrays were incubated with secondary antibody conjugated with 2 nm gold particles (Abcam, U.K.) for 1 hour at room temperature. Then, unbound secondary antibodies were washed away by pipetting on and off with DI water three times. The lipid arrays were ready for imaging by STEM. Au NPs were counted using ImageJ software where Intermodes threshold in the multi-threshold option with a median filter of 2.0 pixel were used to eliminate the background noise before counting the nanoparticles at different magnifications (Figure S4).

### Example 6: EV capture from unpurified samples and EV cargo retention

EVs are abundant in bodily fluids and cell culture medium. Thus, samples can be easily obtained, but these liquids also contain a vast complex background of other components, such as lipids, lipoproteins resembling in size and density the EV proteins and cell debris. Therefore, most EV capturing approaches strictly require pre-purification or pre-enrichment steps, which are time-consuming and expensive. Encouraged by the good performance of our platform in the previous tests, experiments with unpurified cell medium were conducted. Here, MCF7 conditional medium was only pre-filtered with a 0.22 µm pore filter to remove cells or cell debris (>220 nm), which can be achieved in only 30 sec. The filtered medium was then incubated on CD63 AB-carrying lipid microarrays for EV capture without further purification or enrichment steps. Because the EVs were also not fluorescently labelled for these experiments, a fluorescent labeled EpCAM AB was used for immunostaining of the lipid patches after EV capture. With this general setup, a series of different experiments was conducted to evidence the reliable capture of EVs directly out of a complex media background by the lipid microarray platform. EV capture results showing a comparison of unpurified and purified samples are shown in Figure 6. The unpurified MCF7 EVs show an even stronger EpCAM signal than the purified ones, probably because of damage and loss of EVs during the purifying steps. The HT1080 (as expected for being EpCAM negative) shows only a minimal fluorescent signal, caused by some remaining unspecific binding of the EpCAM-AB used for secondary staining. To even further raise sample complexity and coming closer to a situation mimicking clinical samples, purified EVs of MCF7 were spiked into the conditional medium of HT1080 cells (500 µL of purified MCF7 EVs into 12 mL of the conditional medium of HT1080). This results in the breast-cancer-associated EVs being in a vast background of EVs and other biomaterials stemming from non-breast-cancer cells, thus getting closer to a real clinical sample in complexity. Still, the lipid microarrays show a clear signal of captured EpCAM-positive EVs, thus indicating, that the MCF7 EVs could be successfully captured from the spiked sample. The reduced intensity can be understood as caused by the additional dilution and preparation steps during spiking and competitive binding of the HT1080 EVs and MCF7 EVs onto the CD63 AB carrying lipid microarrays. To further ensure that the interpretation of the binding experiments is correct and no unspecific interference of other components in the medium occurs, additional control experiments were implemented (Figure S6). Finally, the aspect of retaining the EV cargo in the system was examined, as the EV content is a rich source of diagnostic and scientific information. It was previously shown, that the content of EVs can be trapped on a surface when a SLM is formed by vesicle fusion of the EVs to the substrate. To elucidate whether the EV cargo is also retained on our platform, a RNA staining dye (SYTO RNASelect, only expressing green fluorescence when bound to RNA) was incubated onto the lipid microarrays after EV capture. Fluorescence signals were obtained from all lipid patches carrying CD63 AB (thus being able to capture EVs), while no fluorescence signal is detected on patches functionalized with IgG1 isotype control AB (Figure 6b). This indicates, that the RNA in the EVs bound to the lipid patches remains trapped in our system, opening up a future downstream analysis, e.g. by targeted retrieval of patches via micropipettes, as previously shown for CTCs.

Purification of extracellular vesicles: MCF7 cells, HT1080 cells and 3T3 fibroblast were cultured in Dulbecco's modified Eagle's medium (DMEM, Life Technologies, Germany) supplemented with 10% exosome-depleted FBS (System Biosciences, U.S.A) under standard cell-culture conditions (37 °C and 5% CO2) for 48 hours. Then, condition medium was centrifuged at 500g for 10 minutes to remove suspended cells. The condition medium was then filtered by 0.22 µm Steriflip^{®} filter (Millipore, U.S.A.) on ice for removing cell debris. In order to do further enrichment of EVs and reduce the amount of the condition medium, it was centrifuged with Amicon^{®} 100 kDa cut-off Ultrafilter (Millipore, U.S.A.) at 5000xg for 30 minutes. The filtered medium was discarded and the EVs were fully recovered by using 200 mL of cold PBS into the filter device to sweep the filter membranes. The enriched EVs sample was stained with the PKH dye (Sigma-Aldrich, U.S.A.) which is a lipophilic fluorescent dye, and then centrifuged again at 5000xg for 30 minutes for removing unbounding dyes. All centrifuge and filtering steps were performed at 4 °C. The labeled EVs were fully recovered from the spin column and loaded into a qEVoriginal SEC column (IZON, U.K.). Fractions of 500 µL each were collected according to the recommendations of the supplier (meaning that the first 3 mL void volume were discarded as the EV fraction is expected to appear at (3.75 ± 0.25) mL. "Fraction 1" corresponds to the first 500 µL sample collected after the void volume passed the column. Additional fractions refer to the subsequent volumes of 500 µL passing, respectively. SEC was done at room temperature, collected fractions were immediately transferred on ice or stored at -80°C after collection.

Capture of EVs from unpurified condition medium: After incubating 48 hours, the condition medium was centrifuged at 500xg for 10 minutes in order to get rid of the big projects (suspensive cells, cell debris etc.) and subsequently filtered by 0.22 µm Steriflip^{®} filter on ice. The prepared EV chip was directly immersed in the filtered medium in a petri dish (VWR, U.S.A) at 4 °C for 1 hour. The chip was washed three times by dipping into 100 mL DI water after incubation and imagined with a fluorescence microscope.

Spiking experiment: The MCF7 EVs were purified by SEC column (qEV35original, IZON LtD) as described above. 500 µL of purified MCF7 EVs was spiked into 12 mL of the condition medium of HT1080 which was cultured for 48 hours. The condition medium of HT1080 was centrifuged at 500xg for 10 minutes and filtered by 0.22 um PES filter (Millipore, U.S.A) in advance. The purified MCF7 EVs and HT1080 condition medium were mixed well and poured onto functionalized EV chips placed in a petri dish on ice for 1 hour. After capturing, the EV chips were washed with DI water (three times by dipping into 100 mL) and then analyzed via optical microscopy.

Optical Microscopy: The images were recorded by Upright microscope (Eclipse 80i, Nikon Instruments Europe B.V., Germany) using onboard software (NIS-Elements, Nikon, Germany). Illumination for fluorescence microscopy was an Intensilight (Nikon, Germany) and filter cubes for TexasRed (excitation/emission wavelength: 559 nm/630 nm, color-coded red) FITC (475 nm/530 nm, color-coded green), and Cy5 (604 nm/712 nm, color-coded purple) were used.

Immunostaining: 0.5 µg of primary anti-EpCAM antibody (Rabbit polyclonal, Abcam, U.K.) admixed with 50 µL of 10% BSA in PBS was incubated on the captured EV chip at 4 °C for 1 hour and washed three times by dipping into 100 mL of DI water. Then, 0.5 µg of secondary anti-rabbit IgG Alexa Fluor 647 (donkey polyclonal, Abcam, U.K.) mixed with 50 µL of 10% BSA in PBS was incubated at 4 °C for 1 hour and then washed three times by dipping into 100 mL DI water, and imagined with a fluorescence microscope.

### Example 7: EV capture from patient sample

To validate our platform further we strived to demonstrate detection from EVs isolated from blood samples of cancer patients. For this, serum was collected from three pancreatic cancer patients and EVs were enriched using SEC (qEV35 nm columns). All fractions were characterized by NTA; BCA and then incubated on lipid patch arrays functionalized with CD63 AB. Successful capture of EVs was confirmed by subsequent staining with EpCAM AB, recognizing cancer-derived EVs (Figure 7b). In all three patients, EVs could successfully be detected on the CD63 functionalized patches by EpCAM immunostaining, while no signal was obtained from negative control fractions (Figure S7). To test if EV nucleic acid cargo is retained on the lipid array, we stained for RNA (Figure 7b, Figure S8). The staining is supporting the assumption of EV cargo being retained, further corroborating the data on RNA retention in the previous section. Overall, the successful capture of tumor-associated EVs from the patient samples underlines the potential of our platform for clinical applications.

Fluorescent labeling of RNA: SYTO RNASelect^{™} Green Fluorescent Cell Stain (5 mM, Thermo Fisher Scientific, U.S.A.) was first diluted to 5 nM into PBS then immediately incubated onto captured-EV chips for 20 minutes at room temperature. After incubation, the EV chips were washed by incubation with 100 µL of DI water two times for 5 minutes each. Then, the EV chips were imagined with fluorescent microscopy.

Detection of tumor-associated EVs from patient samples: Serum samples were collected from pancreatic cancer patients with approval of the local ethic committee. The serum samples were centrifuged 1650xg for 15 minutes according to the recommendations of the vacutainer supplier. The platelets-free serum was transferred into fresh tubes and frozen at -80 °C. 500 µL of cell-free serum were subjected to SEC, using qEV35 original columns (IZON, France). For each patient, 7 subsequent fractions (Fraction 1 to 7) of 500 µL each were collected after the void volume of 3 mL has passed the column. Also 500 µL of the void volume was sampled (Void) as control. 80 µL of each fraction was loaded onto prepared EV chips and incubated for 1 hour at 4 °C. The EV chip was washed three times by dipping into 100 mL of DI water after incubation and then immunostained as described above. For two patient samples, RNA staining was performed as described above. After staining procedures, samples were imaged with fluorescence microscopy.

## Claims

1. A method for isolating extracellular vesicles (EVs) from a sample, comprising the step of contacting the sample with a supported lipid membrane (SLM) capable of binding the EVs.

2. The method according to claim 1, wherein the SLM is placed on a microfluidic chip.

3. The method according to any of claims 1 to 2,
i) wherein the SLM comprises capturing molecules capable of binding the EVs and/or
ii) wherein the capturing molecules are selected from the group consisting of a biotinylated antibody, an unbiotinylated antibody, or a tagged antibody, preferably as HIS-tagged antibody and/or
iii-1) wherein the capturing molecule is a biotinylated antibody bound to streptavidin comprised in the SLM, or
iii-2) wherein the capturing molecule is an unbiotinylated antibody bound to Protein A or Protein G, comprised in the SLM.

4. The method according to any of claims 1 to 3, wherein the EVs are extracellular tumor-associated vesicles.

5. The method according to any of claims 1 to 4,
i) wherein the sample is a body fluid, preferably wherein the sample is blood and/or
ii) wherein the sample is passed microfluidicly across the SLM.

6. The method according to any of claims 1 to 5, wherein the EVs have a diameter of between 20 nm - 800 nm, preferably wherein the EVs have a diameter of between 40 nm - 500 nm.

7. The method according to any of claims 1 to 6, further comprising the step of identifying a molecule comprised in the EVs, preferably wherein the molecule is a DNA molecule, an RNA molecule, a protein, a peptide and/or a lipid molecule.

8. The method according to claim 7, wherein the EVs are taken up with the SLM, preferably wherein the molecule comprised in the EVs is analyzed spectroscopically, preferably by Raman spectroscopy or by TIRF spectroscopy or by ATR spectroscopy.

9. The method according to claim 7, wherein the EVs are not taken up with the SLM.

10. The method according to any of claims 8 or 9, wherein the molecule comprised in the EVs is analyzed by staining and fluorescence detection.

11. The method according to any of claims 7 to 10, wherein the molecule comprised in the EVs is further isolated for further analysis, preferably wherein the molecule is further analyzed by PCR, HPLC, ESI-MS, MALDI-MS or proteomics.

12. A method for diagnosing a tumor, comprising the steps of
i) performing the method according to any of claims 1 to 6,
ii) identifying a molecule comprised in the EVs as a tumor molecule according to any of claims 7 to 11, thereby diagnosing the tumor.

13. A microfluidic chip comprising an SLM for isolating EVs, wherein the microfluidic chip is having the features as defined in claim 3, preferably wherein the SLM is attached via physisorption to the microfluidic chip.

14. The microfluidic chip according to claim 13, wherein the surface opposite to the SLM comprises Polysiloxan (PS) and/or Polydimethylsiloxan (PDMS) and/or glass.

15. Use of a microfluidic chip according to any of claims 13 to 14 for isolating EVs, wherein the isolating comprises the method according to any of claims 1 to 6.
